# EUROPEAN PATENT SPECIFICATION

(11) **EP 1 799 289 B1**
(45) Date of publication and mention of the grant of the patent: **02.05.2012**
(21) Application number: 04787593.5
(22) Date of filing: 16.09.2004
(51) Int. Cl.: A61M 15/00

(54) **Disposable monodose inhaler for powdered medicaments**
Einweg-Monodosis-Inhalator für pulverförmige Medikamente
Inhalateur monodose jetable pour medicaments en poudre

(43) Date of publication of application: 27.06.2007
(73) Proprietor: Cossi, Giampiero, 06063 Magione (PG) (IT)
(72) Inventor: COSSI, Giampiero, I-00151 Roma RM (IT); LA BARBERA, Mario, I-90017 Santa Flavia PA (IT); CECCHINI, Marco, I-06124 Perugia (IT)
(74) Representative: Pizzoli, Antonio
(86) International application number: PCT/IT2004/000501
(87) International publication number: WO 2006/030459

(56) References cited:
- US-A- 5 301 666
- US-A- 5 435 297
- US-A- 5 669 378

## Description

The present invention relates to inhalers for powdered medicaments, and in particular to a disposable monodose inhaler.

It is known that the administration of medicaments in the form of powders to be inhaled is an effective and non-invasive manner to provide a patient with the drug he requires. To this purpose, several devices, generally in the form of reusable multidose inhalers, have been designed to administer powdered medicaments. An example of such an inhaler is disclosed in US 5301666.

This type of inhaler, however, has various drawbacks: a rather high cost; a significant complexity that makes its use rather difficult, in particular for elderly patients; a great difficulty in maintaining it in a satisfactory hygienic state over time; the risk that the powdered medicament is not properly delivered due to thickening or formation of a film caused by humidity or by other factors; the fact that usually most of the medicament does not reach the patient's lungs because it is deposited on the walls of mouth and throat.

In an attempt to overcome these drawbacks there was developed a monodose inhaler, disclosed in US 5669378, that has a much simpler and cheaper structure and can therefore be used as a disposable inhaler. In this way there are no risks of poor hygiene and deterioration of the medicament, neither a significant difficulty in use.

In fact this type of inhaler essentially consists of a pipe-shaped body with a first portion (element), for housing a medicament-containing capsule, connected through a screen to a second portion (element), for delivering the powder, that the patient puts in his mouth. In order to release the powder from the capsule there is provided a button with a spike suitable to pierce the capsule placed in an adequate supporting member, a button-supporting spring being arranged between the button and the capsule-supporting member to prevent a premature piercing of the capsule.

Although this disposable monodose inhaler that represents the closest prior art is a significant improvement with respect to previous multidose inhalers, yet it is also not free from drawbacks.

First of all, it does not solve the problem of the medicament being deposited in the oropharingeal cavity before reaching the lungs. As a consequence, if the patient does not receive the desired relief that he expects from the medicament, he tends to administer himself another dose prematurely with the risk of various unpleasant side effects caused by the overdose. Moreover, the medicament deposited in the oropharingeal cavity can cause some problems such as soreness, coughing, dryness and the like.

Secondly, it still requires assembling the above-mentioned three members (button, spring, capsule support) in the housing portion (element), which implies a certain manufacturing cost as well as the risk of a malfunction in case of defective assembly.

Therefore the object of the present invention is to provide a disposable monodose inhaler which is free from said drawbacks. This object is achieved by means of a monolithic inhaler provided with a bottom duct in the delivering portion (element) and suitable to house an autoperforating cartridge.

The main advantage of the present inhaler is given by the presence of the secondary stream, delivered from the bottom duct, that supports and directs the primary stream, which carries the powdered medicament, so that the primary stream can climb over the patient's tongue. In this way there is prevented the excessive deposition of the medicament in the oropharingeal cavity and there is obtained a greater effectiveness of the treatment thanks to the larger amount of medicament that reaches the lungs.

A further advantage provided by the presence of the secondary stream is the better separation of the drug from the excipient (usually lactose) due to the secondary stream that hits and directs the primary stream.

Still another advantage of this inhaler is its low cost, thanks to the fact that notwithstanding the presence of the bottom duct it can still be manufactured in plastic by injection molding with a monolithic structure. Moreover, this inhaler is suitable to house an autoperforating cartridge, like the cartridge disclosed in WO 03/011708 in the name of one of the inventors, whereby the other members for capsule piercing described in the above-mentioned prior art inhaler can be dispensed with.

Further advantages and characteristics of the inhaler according to the present invention will be clear to those skilled in the art from the following detailed description of an embodiment thereof, with reference to the annexed drawings wherein:

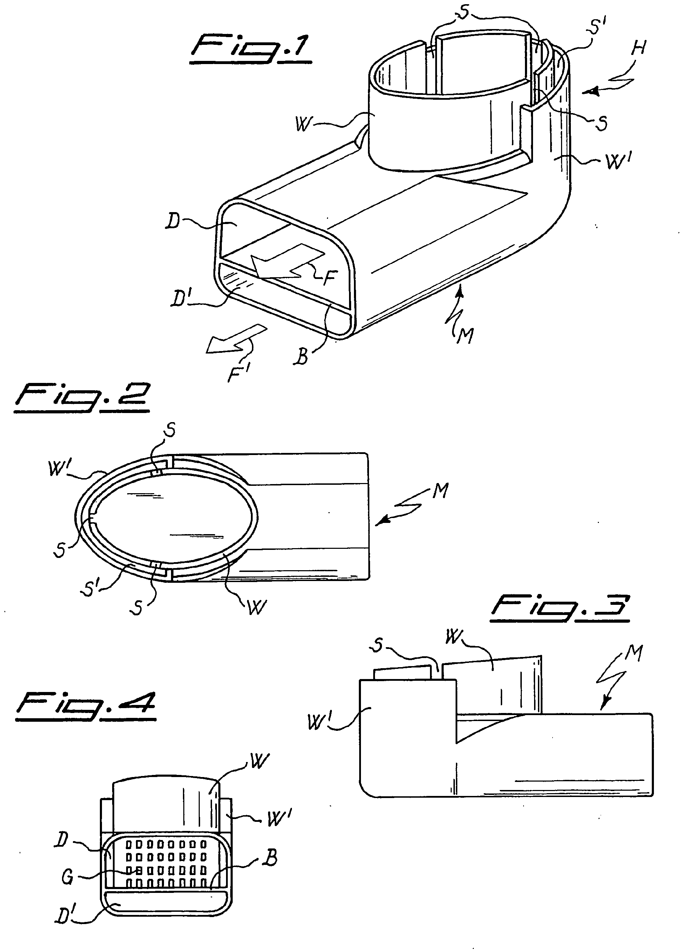

Fig.1 is a front perspective view of the inhaler;
Fig.2 is a top plan view of the inhaler;
Fig.3 is a side view of the inhaler;
Fig.4 is a front view of the inhaler;

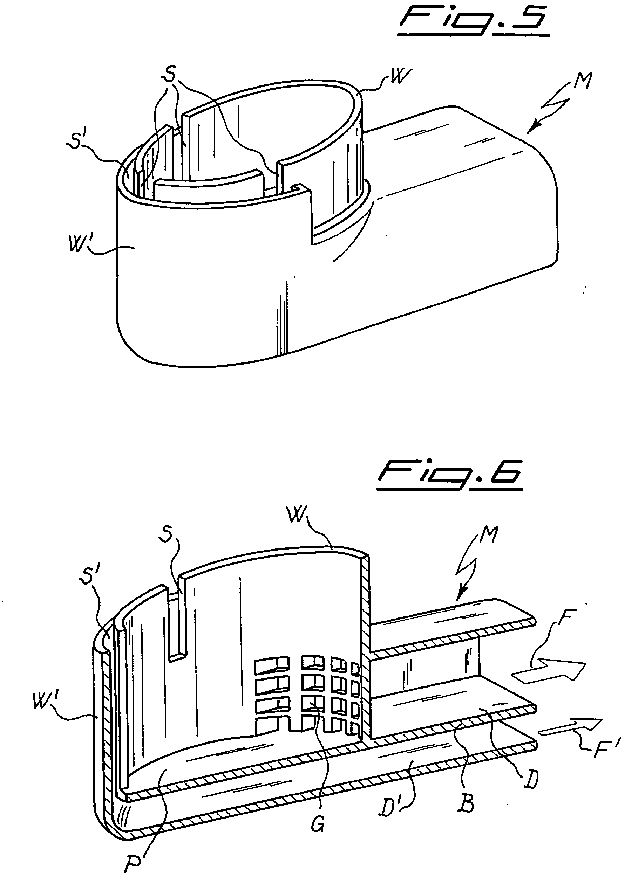

Fig.5 is a rear perspective view of the inhaler; and
Fig.6 is a sectional rear perspective view taken along the longitudinal midplane of the inhaler.

With reference to said figures, there is seen that an inhaler according to the present invention conventionally consists of a hollow substantially smoking pipe-shaped body that has a first portion (element) H, for housing the capsule or cartridge of the powdered medicament, and a second portion (element) M, for delivering the medicament through a delivery duct D whose end is suitable to be placed in the patient's mouth.

In the wall W that defines the housing portion (element) H there are formed one or more vertical slots S to provide air intakes to the inner region P where the powder drops. In the illustrated embodiment there are provided three slots S, namely a central slot extending through the whole height of wall W at the midplane in distal position and other two shorter slots symmetrically arranged with respect to the central slot.

The connection between the powder drop region P and the delivery duct D is achieved through a grid G sized to prevent the passage of pieces of the capsule or cartridge that should drop therefrom upon release of the powder.

The air entering through slots S forms a stream F that carries the powder from region P, through grid G, along the delivery duct D up to the patient's mouth.

The main novel aspect of the present inhaler is that it is designed to generate also a secondary, powderless, stream F' that supports and directs the primary stream F upon inhalation.

To this purpose, in the housing portion (element) H there is provided a second wall W' that encloses wall W at least partially, for example along the rear half of the latter in the illustrated embodiment, and is spaced therefrom so as to obtain an interspace S' between the two walls.

This interspace S' continues below the powder drop region P and extends up to the end of the delivery duct D thus forming a bottom duct D'. In practice, the delivering portion (element) M results therefore consisting of two ducts D, D' one on top of the other separated by a horizontal baffle B.

It is therefore clear that when the patient draws in air to inhale the powder located in region P, where it was released preferably from an autoperforating cartridge as mentioned above, a portion of the air passes through slots S and forms the primary powder-carrying stream F delivered through duct D, while another portion of the air passes through interspace S' and forms the secondary powderless stream F' delivered through the secondary duct D'.

As previously mentioned, the secondary stream F' supports the primary stream F and prevents the powder from depositing, also due to gravity, on the patient's tongue or on other walls of the oropharingeal cavity. This supporting and directing function is particularly important in case the patient holds the distal end of the inhaler too much inclined upwards.

This supporting effect, as well as the other effect of separation of the drug from the excipient, can be increased or decreased by changing the ratio between the cross-sections of ducts D, D' and/or between the cross-sections of the air intakes S, S' or other details. In other words, the two streams F, F' can be adjusted through the design of the various parts of the inhaler in order to obtain several embodiments with different inhalation characteristics for different specific applications.

For example, the air intake S' for the secondary stream F' could be formed directly at the rear end of the secondary duct D', in which case wall W' would extend only below the powder drop region P. Also, the dividing baffle B could be U-shaped rather than plane, so that the "air cushion" formed by the secondary stream F' partially encloses the primary stream F also on the sides.

It is clear that the above-described and illustrated embodiment of the inhaler according to the invention is just an example susceptible of various modifications. In particular, although portions (elements) H, M have been illustrated as connected at 90° it is clear that the monolithic body of the inhaler can be made also with any other angle between said portions from 0° to 90°, e.g. 45° or 60°. Moreover, the number, shape and arrangement of the air intakes to the powder drop region P can be freely changed and therefore be completely different from the above-illustrated slots S.

## Claims

1. Inhaler for powdered medicaments consisting of a hollow substantially smoking pipe-shaped body that has a first element (H), for housing a capsule or cartridge of powdered medicament, defined by a wall (W) in which there are formed one or more air intakes to an inner drop region (P) where the powder drops, and a second element (M) connected substantially perpendicularly to said first element (H) for delivering the medicament by means of a primary stream (F) that carries the powder from said inner drop region (P) along a delivery duct (D) whose end is suitable to be placed in the patient's mouth, **characterized in that** it further includes a secondary duct (D') located under said delivery duct (D) and provided with its own air intake (S') for delivering a powderless secondary stream (F').

2. Inhaler according to claim 1, **characterized in that** in the housing element (H) there is provided a second wall (W') that encloses at least partially the wall (W) and is spaced therefrom so as to obtain an interspace (S') that extends below the inner powder drop region (P) and acts as air intake for the secondary duct (D').

3. Inhaler according to claim 1 or 2, **characterized in that** the air intakes to the inner powder drop region (P) are three slots (S), preferably a central slot extending through the whole height of the wall (W) at the midplane in distal position and other two shorter slots symmetrically arranged with respect to said central slot.

4. Inhaler according to one of the preceding claims, **characterized in that** the dividing baffle (B) that separates the delivery duct (D) from the secondary duct (D') is U-shaped.

5. Inhaler according to one of the preceding claims, **characterized in that** a grid (G) is arranged between the inner powder drop region (P) and the delivery duct (D).

6. Inhaler according to one of the preceding claims, **characterized in that** it is manufactured in plastic by injection molding with a monolithic structure.

7. Inhaler according to one of the preceding claims, **characterized in that** the two elements (H, M) of the body are connected at 90°.

## Patentansprüche

1. Inhalator für pulverförmige Medikamente, der aus einem im Wesentlichen pfeifenförmig geformten Körper besteht, welcher ein erstes Bauteil (H) als Gehäuse für eine Kapsel oder Kartusche mit dem pulverförmigen Medikament aufweist, definiert durch eine Wand (W), in der ein oder mehrere Lufteinlässe für einen inneren Fallbereich (P), wo das Pulver herabfällt, ausgeformt sind, und ein zweites Bauteil (M), das im Wesentlichen im rechten Winkel mit dem ersten Bauteil (H) verbunden ist, zum Zuführen des Medikaments mittels eines Hauptstroms (F), welcher das Pulver aus dem inneren Fallbereich (P) entlang eines Zuführschachtes (D) befördert, dessen Ende zum Einführen in den Mund des Patienten geeignet ist, **dadurch gekennzeichnet, dass** es ferner einen zweiten Schacht (D') aufweist, der unter dem Zuführschacht (D) angeordnet und mit seinem eigenen Lufteinlass (S') versehen ist, um einen pulverfreien Nebenstrom (F') zuzuführen.

2. Inhalator nach Anspruch 1, **dadurch gekennzeichnet, dass** in dem Gehäusebauteil (H) eine zweite Wand (W') vorgesehen ist, die mindestens teilweise die Wand (W) umschließt und von dieser derart beabstandet ist, dass ein Zwischenraum (S') erhalten wird, der sich unterhalb des inneren Pulver-Fallbereichs (P) erstreckt und als ein Lufteinlass für den zweiten Schacht (D') wirkt.

3. Inhalator nach Anspruch 1 oder 2, **dadurch gekennzeichnet, dass** die Lufteinlässe für den inneren Pulver-Fallbereich (P) drei Schlitze (S) sind, vorzugsweise ein zentraler Schlitz, der sich durch die gesamte Höhe der Wand (W) in der Mittelebene in distaler Position erstreckt, und zwei weiteren kürzeren Schlitzen, die in Bezug auf den zentralen Schlitz symmetrisch angeordnet sind.

4. Inhalator nach einem der vorangehenden Ansprüche, **dadurch gekennzeichnet, dass** die Trennwand (B), welche den Zuführschacht (D) von dem zweiten Schacht (D') trennt, eine U-Form aufweist.

5. Inhalator nach einem der vorangehenden Ansprüche, **dadurch gekennzeichnet, dass** ein Gitter (G) zwischen dem inneren Pulver-Fallbereich (P) und dem Zuführschacht (D) angeordnet ist.

6. Inhalator nach einem der vorangehenden Ansprüche, **dadurch gekennzeichnet, dass** er aus Kunststoff mittels Spritzgießen mit einer monolithischen Struktur geformt ist.

7. Inhalator nach einem der vorangehenden Ansprüche, **dadurch gekennzeichnet, dass** die beiden Bauteile (H, M) von dem Körper mit einem Winkel von 90° miteinander verbunden sind.

## Revendications

1. Inhalateur pour médicaments en poudre se composant d'un corps creux sensiblement en forme de pipe qui a un premier élément (H) pour loger une capsule ou cartouche de médicament en poudre, défini par une paroi (W) dans laquelle sont formées une ou plusieurs admissions d'air jusqu'à une région de chute interne (P) où la poudre tombe, et un deuxième élément (M) raccordé de manière sensiblement perpendiculaire audit premier élément (H) pour distribuer le médicament au moyen d'un courant principal (F) qui transporte la poudre de ladite région de chute interne (P) le long d'un conduit de distribution (D) dont l'extrémité est appropriée pour être placée dans la bouche du patient, **caractérisé en ce qu'**il comprend en outre un conduit secondaire (D') positionné sous ledit conduit de distribution (D) et prévu avec sa propre admission d'air (S') pour distribuer un courant secondaire sans poudre (F').

2. Inhalateur selon la revendication 1, **caractérisé en ce que** dans l'élément de boîtier (H), on prévoit une deuxième paroi (W') qui enferme au moins partiellement la paroi (W) et est espacée de cette dernière afin d'obtenir un espace intermédiaire (S') qui s'étend au-dessous de la région de chute de poudre interne (P) et sert d'admission d'air pour le conduit secondaire (D'.

3. Inhalateur selon la revendication 1 ou 2, **caractérisé en ce que** les admissions d'air par rapport à la région de chute de poudre interne (P) sont trois fentes (S), de préférence une fente centrale s'étendant à travers toute la hauteur de la paroi (W) au niveau du plan central dans la position distale et deux autres fentes plus courtes agencées de manière symétrique par rapport à ladite fente centrale.

4. Inhalateur selon l'une quelconque des revendications précédentes, **caractérisé en ce que** le déflecteur de division (B) qui sépare le conduit de distribution (D) du conduit secondaire (D') est en forme de U.

5. Inhalateur selon l'une quelconque des revendications précédentes, **caractérisé en ce qu'**une grille (G) est agencée entre la région de chute de poudre interne (P) et le conduit de distribution (D).

6. Inhalateur selon l'une quelconque des revendications précédentes, **caractérisé en ce qu'**il est fabriqué avec une matière plastique par moulage par injection avec une structure monolithique.

7. Inhalateur selon l'une quelconque des revendications précédentes, **caractérisé en ce que** les deux éléments (H, M) du corps sont raccordés à 90°.
